# EUROPEAN PATENT APPLICATION

(11) **EP 4 477 239 A1**
(43) Date of publication of application: **18.12.2024**
(21) Application number: 23752446.7
(22) Date of filing: 10.02.2023
(51) Int. Cl.: A61L 27/50, A61L 27/58, A61L 27/18, A61L 27/52, A61L 27/20

(54) **COSMETIC PRODUCT CONTAINING STERILE PCL MICROSPHERES**

(30) Priority: 10.02.2022 CN 202210125983
(71) Applicant: Sinclair Pharmaceuticals Limited, Chester CH4 9QT (GB)
(72) Inventor: ZHANG, Xuan, Hangzhou, Zhejiang 310011 (CN); WANG, Bin, Hangzhou, Zhejiang 310011 (CN); ZHANG, Hairu, Hangzhou, Zhejiang 310011 (CN); WANG, Zhuo, Hangzhou, Zhejiang 310011 (CN); XIA, Wenrong, Hangzhou, Zhejiang 310011 (CN); GAO, Jian, Hangzhou, Zhejiang 310011 (CN); DONG, Xijian, Hangzhou, Zhejiang 310011 (CN)
(74) Representative: Nederlandsch Octrooibureau
(86) International application number: PCT/CN2023/075505
(87) International publication number: WO 2023/151668

(57) **Abstract**

The present invention relates to a beauty product containing sterile PCL microspheres, which beauty product contains sterile PCL microspheres. The sterile PCL microspheres are subjected to an irradiation and sterilization treatment, the surface of the sterile PCL microsphere is smooth and spherical, the average particle size is in the range of 26-46 µm, and the roundness is ≥ 0.96. The sterile PCL microspheres are obtained by means of an irradiation and sterilization treatment using a γ or β ray; and a CMC-Na gel is obtained by means of a sterilization treatment using a moist heat sterilization method. The sterile PCL microspheres subjected to irradiation and sterilization, and the CMC-Na gel obtained by means of moist heat sterilization are uniformly mixed and then filled into a syringe sterilized with ethylene oxide, thus forming the sterile beauty product that can not only meet the requirements for sterile injection, but can also better ensure the quality of the product and reduce side effects such as an inflammatory reaction.

## Description

### Technical Field

The present invention relates to the field of medical cosmetology or medical preparations, and specifically to a beauty product containing sterile PCL microspheres, and a preparation method and a sterilization method therefor.

### Background Art

Polycaprolactone (PCL, CAS number: 24980-41-4**),** also referred to as poly ε-caprolactone, is a high-molecular polymer formed by the ring-opening polymerization of an ε-caprolactone monomer under the catalysis of a metal anion complex catalyst, wherein different molecular weights can be obtained by controlling the polymerization conditions. It appears as a white solid powder or particle, and is non-toxic, insoluble in water and easily soluble in a variety of polar organic solvents. PCL has good biocompatibility, good compatibility with organic high polymers and good biodegradability, can be used as a cell-growth-supporting material, can be mutually compatible with a variety of conventional plastics, and can be completely degraded in the natural environment and in vivo. The products of PCL degradation are CO₂ and H₂O, which are non-toxic to humans. PCL is used as a medical beauty filler in an in vivo implant material, and may stimulate collagen production. A PCL-based carboxymethyl cellulose gel, after being injected, can provide an immediate and sustained volumizing effect.

PCL as a filler raw material is usually made into microspheres, the size, shape and smoothness of which have been proven to have a great influence on tissue reaction and to determine the extent of the reaction. When the PCL microspheres are prepared into a regular spherical shape with a smooth surface, inflammation can be minimized; moreover, the smoother the sphere formed, the more conducive it is to hypodermic injection, and highly smooth microspheres can reduce the occurrence of a foreign-body response while stimulating the one-by-one wrapping of fibroblasts under wrinkles.

Injectable beauty products are administered by direct injection into the subcutaneous tissue of human bodies, and if the products carry pathogens on their surfaces or in their interiors, they may result in serious infections and cause serious adverse reactions in patients; therefore, biomedical fillers for human body implantation need to be sterilized. Sterilization is not only required to kill or remove microbial propagules and spores to maximize the safety and efficacy of injectable agents, but also to ensure the morphology, stability and clinical efficacy of injectable beauty products; therefore, it is important to choose an appropriate sterilization method to ensure the quality of beauty products. Over the past few decades, a variety of sterilization methods have been used in the fields of medical device products and medical beauty injection products, including steam treatment, heating/autoclaving sterilization, ethylene oxide treatment, plasma sterilization, and electron beam irradiation (β-irradiation), γ-irradiation, or X-ray irradiation. The sterilization method must be compatible with the choice of material for the implant, so as to avoid the ineffectiveness of the sterilization process and to reduce its destructive effect on the product.

PCL has a relatively low melting temperature (about 60°C) and cannot withstand high temperatures. PCL microspheres will melt at temperatures above 50°C; therefore, for PCL microspheres, steam treatment and autoclaving sterilization techniques are not suitable, and may damage the material or the structure of the implant. Cassan et al. evaluated three low-temperature techniques regarding PCL sterilization, including β-ray irradiation sterilization, γ-ray irradiation sterilization, and X-ray irradiation sterilization, and found that irradiation may lead to a decrease in molecular weight and an increase in crystallinity, and that microspheres are also affected by irradiation, with the morphology irradiation effect yielded being is dose dependent, and higher irradiation doses leading to stronger changes. Of these treatments, it was found that γ irradiation appears to be the least suitable method, while electron beams (β irradiation) have a smaller effect.

In addition, it is highly important to maintain the sphere shape and surface smoothness of the injected microspheres; this can improve the fluidity of the gel and also prevent adverse foreign-body responses. This is because the overall biocompatibility of dermal fillers is determined by the smooth and regularly shaped microparticles, and these smooth microparticles may stimulate collagen growth, whereas rough, irregularly shaped microparticles are surrounded by macrophages and multinucleated giant cells, leading to capsular fibrosis, inhibition of collagen production, and potential adverse foreign-body responses.

In addition, other components of injectable beauty products containing PCL microspheres, the syringe component and the CMC-Na gel, also need to be sterilized. It is known that syringe materials such as polypropylene materials may discolour and become brittle when sterilized by irradiation and are therefore unsuitable for sterilization by γ-irradiation, and EP 3685826 B1 mentions the sterilization of syringes and injection needles with ethylene oxide, hydrogen peroxide, etc. US 10905786 B2 mentions the use of evaporated hydrogen peroxide for sterilizing syringes. As for sterilization methods for CMC gels, WO 2011089173 A1 mentions that the sterilization phase of suspensions containing CMC microspheres usually involves the use of treatment with a back-pressure autoclave, and Tichy et al. evaluated the effects of steam sterilization and the presence of electrolytes on the rheology and textile properties of cellulose derivative-, carrageenan- and xanthan gum-based hydrogels. Moreover, once a syringe is filled with a gel injectable agent, the assembled injectable beauty product can no longer be sterilized with ethylene oxide because ethylene oxide and other gases cannot pass through the syringe. Therefore, how to effectively sterilize PCL microspheres while maintaining the sphere shape and surface smoothness of the microspheres to prepare beauty products containing sterile PCL microspheres is a problem that urgently needs solving in the field of medical cosmetology.

### Summary of the Invention

The present invention relates to a beauty product containing sterile PCL microspheres, which product needs to be injected into human bodies, and therefore, strict sterilization of the product itself is required, in particular the PCL microspheres. By studying the effects of γ-ray and β-ray irradiation sterilization on PCL microspheres, it is found that γ rays have a better sterilization effect than β rays. It is also found that both high doses of γ rays and high doses of β rays adversely affect the PCL microspheres themselves, mainly in terms of changes in the surface smoothness and the shape of the spheres of the PCL microspheres. Therefore, the mode of irradiation and the dose of irradiation will be very important in the sterilization of PCL microspheres.

In the prior art, optical microscopy and electron microscopy are mostly used to observe the surface smoothness and the shape of the spheres of microspheres (e.g., to observe whether the surface of the microspheres is smooth, whether the spheres are round, etc.), which is relatively subjective and does not include a quantitative index, and it is also difficult to achieve real-time monitoring and accurate control in actual production processes. In order to overcome the above problem, in the present invention, the Sysmex 3000 particle morphology and particle size analyser is used in combination with flow particle image analysis (FPIA) technology to detect the roundness of PCL microspheres, which, through quantitative indexes, enables the characterization of the sphere shape of the microspheres, the taking into account of the surface smoothness, and also the carrying out of quality control in actual production, effectively satisfying the quality requirements for sterile PCL microspheres.

The present invention relates to a beauty product containing sterile PCL microspheres, comprising sterile PCL microspheres, wherein the sterile PCL microspheres are sterilized by irradiation, have a smooth surface, are of a spherical shape, and have an average particle size range of **26-46** µm, preferably **28-42** µm, and a roundness of ≥ **0.96,** preferably **0.97** or more, **0.98** or more, or **0.99** or more.

The beauty product as previously described, wherein the sterile PCL microspheres are obtained by means of irradiation sterilization with γ or β rays, preferably by means of γ-ray irradiation sterilization, the irradiation dose is **20** kGy-**30** kGy, and the irradiation dose of **20** kGy, **25** kGy or **30** kGy.

The beauty product as previously described, wherein in the sterile PCL microspheres, the microspheres with a particle size of **25-50** µm have a percentage of ≥ **65%,** the microspheres with a particle size of less than **25** µm have a percentage of ≤ **20%,** and the microspheres with a particle size of more than **50** µm have a percentage of ≤ **15%;** preferably, the microspheres with a particle size of **25-50** µm account for ≥ **70** %, microspheres with a particle size of less than **20** µm account for ≤ **1%,** microspheres with a particle size of **20-25** µm account for ≤ **15%,** and the microspheres with a particle size of more than **50** µm account for ≤ **14%.** Microspheres with the above particle size distribution can significantly reduce the incidence of foreign body granulomas and improve the biosafety of beauty products.

The beauty product as previously described, wherein the sterile PCL microspheres have a weight-average molecular weight of **8,000-80,000** Da, for example **10,000** Da, **20,000** Da, **30,000** Da, **40,000** Da, **60,000** Da and **80,000** Da, and preferably **10,000-40,000** Da. The polydispersity index is **1.5-3.0,** and preferably **1.8-2.5.** The beauty products using the above weight-average molecular weight have a degradation time in human bodies of more than **12** months, **18** months, **24** months, and **48** months.

The beauty product as previously described, wherein the change rate of the roundness of the PCL microspheres before and after irradiation is ≤ **3.5%,** preferably ≤ **3%, 2.5%** or **2%.**

The beauty product as previously described, further comprising a CMC-Na gel resulting from a sterilization treatment by means of a moist heat sterilization method.

The beauty product as previously described, further comprising a sterilized syringe.

A method for preparing the sterile PCL microspheres of claim **1,** wherein the specific steps for obtaining sterile PCL microspheres by means of irradiation sterilization are as follows:
step **1:** weighing an appropriate amount of PCL microspheres and placing same in a centrifuge tube, and marking the irradiation dose and the type of microspheres on the centrifuge tube;
step **2:** in order to ensure a sterile state and no leakage of samples during detection, sealing the centrifuge tube with a sealing film;
step **3:** putting the centrifuge tube into a packaging box, and pasting a label on the outside of the box to label information about the sterilization dose; and
and step **4:** receiving irradiation with γ rays or β rays.

The method as previously described, wherein the irradiation dose is **20** kGy, **25** kGy or **30** kGy.

A method for preparing the PCL microspheres as previously described, wherein the specific steps are as follows:
dissolving a PCL material in DCM to form an oil phase; dropwise adding the oil phase to an MC solution under stirring, subjecting same to high-speed shearing by means of a high-speed shear mixer, and stirring same for **2-4** h; and subjecting same to washing with water and centrifuging 3 times, followed by screening and drying to obtain the product, wherein the ratio of the oil phase to an aqueous phase is **1 : 5** to **1 : 50,** preferably **1 : 8-1 : 30,** and more preferably **1 : 10-1** : **20;** and the mass concentration of polycaprolactone in the oil phase is **10** mg/ml to **50** mg/ml, preferably **15 mg/ml-30** mg/ml, and more preferably **15** mg/ml-**25** mg/ml.

The method as previously described, wherein for PCL having a weight-average molecular weight of **40,000-80,000,** after the step of high-speed shearing, there is also a step of ultrasonic treatment; and the ultrasonic treatment is performed for **3-6** min.

The method as previously described, wherein the high-speed shearing is performed at a rotational speed greater than **12,000** r/min for **1-5** min.

The method as previously described, wherein the stirring is performed at a rotational speed of **800-1,200** r/min for **2-4** h.

A method for preparing the beauty product containing sterile PCL microspheres as previously described, wherein the method comprises a step of mixing sterile PCL microspheres with a CMC-Na gel resulting from sterilization by means of a moist heat sterilization method until uniform.

The preparation method as previously described, wherein the uniform mixing step comprises: performing uniform mixing under vacuum conditions.

The preparation method as previously described, wherein the uniform mixing process is performed by using a planetary agitator or a vacuum mixing and defoaming machine.

A method for preparing the CMC-Na gel as previously described, wherein the specific steps are as follows:
step **1:** transferring the manufactured gel to a container, and placing same in an autoclave;
and step **2:** performing steam sterilization by using a high-pressure steam sterilization method, wherein the steam pressure is **101-105** kPa, the temperature is **119-123°C,** for **15-20** min.

A method for preparing the beauty product as previously described, wherein the specific steps are as follows:
step **1:** performing irradiation sterilization to obtain sterile PCL microspheres; performing sterilization by means of a moist heat sterilization method to obtain a CMC-Na gel; and sterilizing a syringe with ethylene oxide;
step **2:** adding the sterile PCL microspheres resulting from the sterilization to the CMC-Na gel and mixing same until unform;
and step **3:** packaging the uniformly mixed CMC-Na gel loaded with the sterile PCL microspheres into the syringe.

The beneficial technical effects of the present invention are as follows:
the colony count detection value of the sterile PCL microspheres obtained by means of a certain dose of γ- or β-ray irradiation can reach a "not detected" level; especially when γ or β rays **of 20 kGy-30kGy** are used to irradiate PCL microspheres with a weight-average molecular weight of **10,000-40,000** Da, it can be seen by electron microscope photographs that their surfaces have fewer depressions and grooves, so that the surface smoothness and the roundness of the PCL microspheres are less affected, and skin granulomas and inflammatory responses are less likely to be induced. The sterile PCL microspheres subjected to irradiation and sterilization, and the CMC-Na gel obtained by means of moist heat sterilization are uniformly mixed and then filled into a syringe sterilized with ethylene oxide, thus forming the sterile beauty product that can not only meet the requirements for sterile injection, but can also better ensure the quality of the product and reduce side effects such as an inflammatory reaction.

### Brief Description of the Drawings

In FIG. **1****,** a and b show a scanning electron microscope photograph and a microscope photograph of PCL microspheres in Example 1, respectively.
FIG. **2** shows a scanning electron microscope photograph of PCL microspheres having a weight-average molecular weight of **10,000** Da.
In FIG. **3****,** a to d show electron microscope photographs of PCL microspheres having a weight-average molecular weight of **10,000** Da after being sterilized by γ rays at **15, 20, 30** or **40** kGy.
FIG. **4** shows an electron microscope photograph of PCL microspheres having a weight-average molecular weight of **40,000** Da after being sterilized by γ rays at **30** kGy.
In FIG. **5****,** a to b show electron microscope photographs of PCL microspheres having a weight-average molecular weight of **10,000** Da after being sterilized by β rays at **30** or **40** kGy.

### Detailed Description of the Embodiments

The present invention is further described below by way of examples and comparative examples, but is not limited thereto.

### Example 1: Preparation of PCL microspheres

Raw materials: **500** mg each of a PCL material having a weight-average molecular weight of **10,000** Da and a PCL material having a weight-average molecular weight of 40,000 Da, **2.5** mL of DCM, and **25** mL of 1% MC solution.

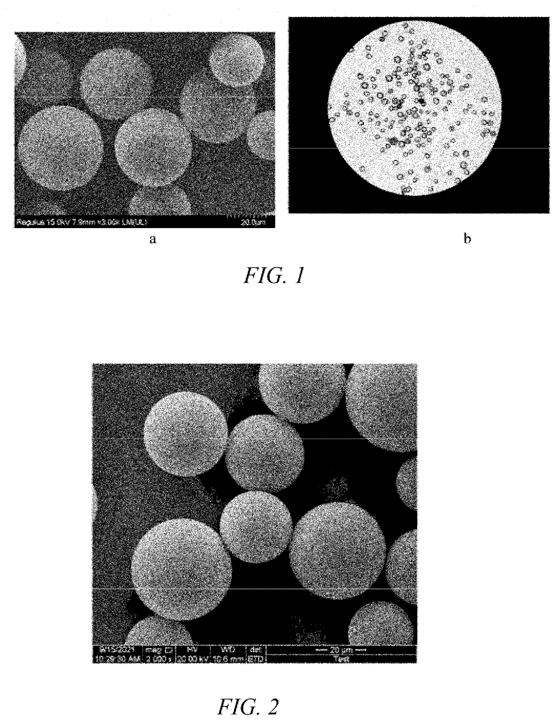

Steps: subjecting **500** mg of the PCL material to vortex dissolution in **2.5** ml of DCM to form an oil phase; dropwise adding the oil phase into **25** ml of **1%** MC solution while stirring, then further stirring same for **30** s, and then shearing same by using a high-speed shear mixer (DLAB **D-160)** at gear **2 (14,000** r/min) for **1** min, subjecting same to an ultrasonic treatment for **5** min and stirring **(1000** r/min) same for **3** h; subjecting same to washing with water and centrifuging **(10,000** r/min) **3** times, **15** min each time, followed by screening and drying for **24** h. The prepared PCL microspheres were of a uniform size and a suitable particle size. FIG. **1**a and FIG. 1b show a scanning electron microscope photograph and a microscope photograph (microscope **BM200010x)** of the PCL microspheres, respectively.

### Example 2: Detection of PCL microspheres before sterilization

The PCL microspheres prepared by using the method of Example 1 were firstly observed under a microscope, and it was found that the prepared PCL microspheres had a uniform and suitable particle size (see FIGS. 1a and 2).

The particle size, polydispersity index, crystallinity, roundness and colony count of PCL microspheres of 10,000 Da and 40,000 Da before and after sterilization were detected in the present invention. The particle size range of the PCL microspheres was detected by using a morphological particle size analyser (Topsizer Plus); the polydispersity index of the PCL microspheres was detected by using gel permeation chromatography (GPC); the crystallinity of the PCL microspheres was detected by using differential scanning calorimetry (DSC); the roundness and the change rate of the PCL microspheres were detected by using flow particle image analysis (FPIA) technology; and the colony count of the PCL microspheres before and after sterilization was detected by using a colony counting method.
**(1)** Detection of particle size: The particle size range of the PCL microspheres was detected by using a morphological particle size analyser, wherein the procedure is as follows:
   weighing **0.1** g of the microspheres and placing same in a centrifuge tube, adding **10** ml of water, oscillating and dispersing the resultant mixture, subjecting same to an ultrasonic treatment for **30** min, and then dispersing same into a beaker filled with **500** mL of deionized water in advance, and determining the particle size by using an OMEC particle size instrument three times and taking the average value.
   It was found after the detection and analysis that the PCL microspheres having a weight-average molecular weight of **10,000** Da have, before sterilization, an average particle size of **35.40** µm, and PCL microspheres with a particle size of less than **20** µm account for **0.66%,** PCL microspheres with a particle size of **20-25** µm account for **18.77%,** PCL microspheres with a particle size of **25-50** µm account for **65.60%,** and PCL microspheres with a particle size of more than **50** µm account for **14.97%;** the PCL microspheres having a weight-average molecular weight of **40,000** Da have, before sterilization, an average particle size of **37.12** µm, and PCL microspheres with a particle size of less than **20** µm account for **0.62%,** PCL microspheres with a particle size of **20-25** µm account for **14.73%,** PCL microspheres with a particle size of **25-50** µm account for **70.67%,** and PCL microspheres with a particle size of more than **50** µm account for **13.98%.**
**(2)** Detection of polydispersity index: the polydispersity index of the PCL microspheres was detected by using gel permeation chromatography (GPC), wherein the specific detection steps are as follows:
   Instrument model: Agilent **1260** Infinity
   Detector: Agilent **1260** RID
   Chromatographic column: Waters Styragel HR **5E (7.8*300** mm, **2000-400** w) and Waters Styragel HR **2 (7.8*300** mm, **500-2** w), in series
   Column temperature: 35°C
   Software: ChemStation for LC systems
   Standard: PS (polystyrene);
   weighing a certain amount of PCL microspheres, dissolving same in a tetrahydrofuran solvent and filtering same, injecting same into an sample inlet by using a sample injection needle after the instrument is stabilized, rinsing the chromatographic column to obtain a complete GPC curve, and calculating the molecular weight and the polydispersity index thereof according to the detected GPC curve.
   It was found after the detection and analysis that the PCL microspheres having a weight-average molecular weight of **10,000** Da have, before sterilization, a polydispersity index (PDI) of: **2.1652;** and the PCL microspheres having a weight-average molecular weight of **40,000** Da have, before sterilization, a polydispersity index (PDI) of: **1.8001.**
(3) Detection of crystallinity: the crystallinity of the PCL microspheres was detected by using differential scanning calorimetry (DSC), wherein the specific detection steps are as follows:
   firstly heating the PCL microspheres to **80°C** by using a differential scanning calorimeter (CED-E-DSC-**045**D SC**250**) and maintaining the temperature for **10** min, and then cooling same to **-40°C** at the same rate and under the protection of nitrogen, recording the temperature increase scanning curve from **-40°C** to **80°C,** the temperature increase rate being **10°C/min,** and the crystallinity being calculated by a specific melting enthalpy method: f = ΔHₘ/ΔH_{m,c}, where ΔHₘ is the melting enthalpy obtained by calculating the peak area of the DSC analytical curve, and ΔH_{m,c} is the melting enthalpy of **100%** crystallized PCL, which was calculated based on the value of **139.5** J/g reported in the literature. The detection results are: the PCL microspheres having a weight-average molecular weight of **10,000** Da have a crystallinity of **70.2266%;** and the PCL microspheres having a weight-average molecular weight of **40,000** Da have a crystallinity of **64.9681%.**
**(4)** Detection of roundness and change rate thereof: the roundness of the microspheres was detected by using a Sysmex **3000** particle morphology and particle size analyser (Malvern Instruments, UK) in combination with flow particle image analysis (FPIA) technology. The specific steps are:
   dispersing PCL microspheres in phosphate-buffered saline (PBS) to form a diluted dispersion which is suitable for FPIA analysis; taking two-dimensional pictures of nearly a thousand individual particles by using flow particle image analysis software as they pass through a flow cell of the Sysmex **3000** particle morphology and particle size analyser; calculating the two-dimensional area of the particles through FPIA, and generating a perfect circle having the same two-dimensional area; and then calculating the circumference of the perfect circle by means of the software, and comparing same with the actual total length of the edge of a particle. Roundness is the ratio of the circumference of the perfect circle to the actual total length of the edge of the particle. The ratio is 1 if the specimen particle is a perfect circle. The more irregular the particle shape and the less smooth the surface, the more the ratio becomes less than 1. The average roundness of the specimens calculated by this method always lies between 0 and 1. The software will automatically calculate the average roundness of 1,000-2,000 microspheres in one test and calculate the average value. After the detection, the PCL microspheres of **10,000** Da and **40,000** Da both have a roundness of more than 0.99 (reaching **0.9951** and **0.9967,** respectively).
   The roundness change rate is the ratio of the roundness change before and after sterilization to the roundness before sterilization: (roundness before sterilization-roundness after sterilization) / roundness before sterilization.
(5) Method for detecting colony count:
   Step **1:** dilution of sample. A certain amount of a PCL microsphere sample is weighed and placed into a sterile homogenizing cup containing a phosphate buffer solution or saline, and homogenized at **8,000** r/min-**10,000** r/min for **1 min-2** min; a **1 : 10** homogenized sample solution is taken by suction by using a sterile suction tube or a micropipette, slowly injected into a sterile test tube containing a diluent along the wall of the tube (care being taken that the tip of the suction tube or pipette tip does not touch the surface of the diluent), the test tube is shaken or repeatedly blown by using another sterile suction tube to mix same until uniform, so as to prepare a **1 : 100** homogenized sample solution; for each incremental dilution, the sterile suction tube or pipette tip is changed once; **2-3** homogenized sample solutions (liquid samples may include the stock solution) at appropriate degrees of dilution are selected based on an estimate of the contamination status of the sample; for **10-**fold incremental dilution, **1** mL of a homogenized sample solution is taken by suction into a sterile flat plate, and two flat plates are made for each degree of dilution; **1** mL of a blank diluent is further respectively taken by suction and added into two sterile flat plates as a blank control; **15 mL-20** mL of a plate count agar culture medium cooled to **46°C** (which can be kept in a thermostatic water bath box at **46°C** ± **1°C** for temperature maintenance) is poured into a flat plate in a timely manner and the flat plate is rotated to mix the materials until uniform.
   Step **2:** Culture. The plate is turned over after the agar solidifies, and same is cultured at **36°C** ± **1** for **48** h ± **2** h.
   Step **3:** Colony counting. Colony count is expressed by colony-forming units (CFU). Plates with a colony count between **30** CFU - **300** CFU and without spreading colony growth were selected for counting the total number of colonies. For plates with less than **30** CFU, the specific colony count was recorded, and the plates with more than **300** CFU could be recorded as "too many to count". The colony count for each degree of dilution should be the average of two plates. It was known from the colony counting that colonies were detected in both PCL microspheres having a molecular weight of **10,000** Da and PCL microspheres having a molecular weight of **40,000** Da before sterilization, and the colony count is < **100** (grade).

### Example 3: Sterilizing PCL microspheres by using γ rays

The specific steps for the irradiation sterilization of PCL microspheres are as follows:
Step **1:** weighing an appropriate amount of microspheres and placing same in a centrifuge tube, and marking the irradiation dose and the type of microsphere on the centrifuge tube;
Step **2:** in order to ensure a sterile state and no leakage of samples during detection, sealing the centrifuge tube with a sealing film;
Step **3:** putting centrifuge tubes that are due to be sterilized and which have the same irradiation dose in a packaging box for, and pasting a label on the outside of the box to label information about the sterilization dose; and
Step **4:** the packages of encapsulated PCL microspheres receiving different doses of γ-ray **(Co60)** irradiation in groups.

Four doses of **15, 20, 30, 40** kGy and others were selected for irradiation, and the irradiated PCL microspheres were further detected and analyzed, wherein the detection methods and steps are as shown in Example **2.**

The detection results are as follows:

**Table 1: Sterile PCL microspheres having a weight-average molecular weight of 10,000 Da**

| **Irradiation dose** | **Colony count** | **Particle size range** | **Polydispersit y index PDI** | **Crystallinity** | **Roundness** | **Roundness change rate** |
|---|---|---|---|---|---|---|
| **15** kGy | <**5** | Average particle size: **35.33** µm; | **2.1726** | **74.3774%** | **0.9892** | **0.59%** |
| | | Microspheres of less than **20** µm account for **0.71%** | | | | |
| | | Microspheres of **20-25** µm account for **18.79%** | | | | |
| | | Microspheres of **25-50** µm account for **65.63%** | | | | |
| | | Microspheres of more than **50** µm account for **14.87%** | | | | |
| **20** kGy | Not detected | Average particle size: **35.22** µm; | **2.1787** | **74.7471%** | **0.9765** | **1.87%** |
| | | Microspheres of less than **20** µm account for **0.77%** | | | | |
| | | Microspheres of **20-25** µm account for 18.68% | | | | |
| | | Microspheres of **25-50** µm account for **66.02%** | | | | |
| | | Microspheres of more than **50** µm account for **14.53%** | | | | |
| **30** kGy | Not detected | Average particle size: **35.14** µm; | **2.1816** | **75.5367%** | **0.9675** | **2.77%** |
| | | Microspheres of less than **20** µm account for **0.83**% | | | | |
| | | Microspheres of **20-25** µm account for **18.65**% | | | | |
| | | Microspheres of 25-50 µm account for **66.43**% | | | | |
| | | Microspheres of more than **50** µm account for **14.09%** | | | | |
| **40 kGy** | Not detected | Average particle size: **35.05** µm; | **2.1950** | **77.2368%** | **0.9547** | **4.06%** |
| | | Microspheres of less than **20** µm account for **0.89**% | | | | |
| | | Microspheres of **20-25** µm account for **18.72**% | | | | |
| | | Microspheres of **25-50** µm account for **66.52**% | | | | |
| | | Microspheres of more than **50** µm account for **13.87**% | | | | |

**Table 2: Sterile PCL microspheres having a weight-average molecular weight of 40,000 Da**

| **Irradiation dose** | **Colony count** | **Particle size range detected after sterilization** | **Polydispersit y index PDI** | **Crystallinity** | **Roundness** | **Roundness change rate** |
|---|---|---|---|---|---|---|
| **15** kGy | <**5** | Average particle size: **37.07** µm; | **1.8573** | **69.4684%** | **0.9855** | **1.12%** |
| | | Microspheres of less than **20** µm account for **0.75**% | | | | |
| | | Microspheres of **20-25** µm account for **14.71%** | | | | |
| | | Microspheres of **25-50** µm account for **70.65%** | | | | |
| | | Microspheres of more than **50** µm account for **13.89%** | | | | |
| **20 kGy** | Not detected | Average particle size: **36.91** µm; | **1.8786** | **70.2789%** | **0.9743** | **2.25%** |
| | | Microspheres of less than **20** µm account for **0.81**% | | | | |
| | | Microspheres of **20-25** µm account for **14.82%** | | | | |
| | | Microspheres of **25-50** µm account for **70.74**% | | | | |
| | | Microspheres of more than **50** µm account for **13.63%** | | | | |
| **30 kGy** | Not detected | Average particle size: **36.82** µm; | **1**.**8925** | **70.7787%** | **0.9618** | **3.50%** |
| | | Microspheres of less than **20** µm account for **0.89%** | | | | |
| | | Microspheres of **20-25** µm account for **14.91%** | | | | |
| | | Microspheres of **25-50** µm account for **70.99%** | | | | |
| | | Microspheres of more than **50** µm account for **13.21%** | | | | |
| **40kGy** | Not detected | Average particle size: **36.79** µm; | **1.9117** | **74.4983%** | **0.9501** | **4.68%** |
| | | Microspheres of less than **20** µm account for **0.96%** | | | | |
| | | Microspheres of **20-25** µm account for **14.83%** | | | | |
| | | Microspheres of **25-50** µm account for **71.34%** | | | | |
| | | Microspheres of more than **50** µm account for **12.87%** | | | | |

It can be seen from the above results that the colony count of the PCL microspheres is significantly reduced after γ-ray irradiation sterilization, which indicates that γ-ray irradiation can effectively sterilize PCL microspheres.

It can be seen from the comparison of the experimental results of each dose of γ rays that when the PCL microspheres are sterilized by irradiation with γ rays at **15** kGy, the colony count (grade) thereof drops to <5; although the colony count is already low, for a beauty product that is injected into human skin, bacterial contamination will have a serious impact on the quality of the product, and an incompletely sterilized product may lead to serious inflammatory responses. Therefore, the colony count needs to be controlled to a standard of "not detected". It can be seen from Tables **1-2** that when the dose of γ rays reaches **20** kGy or more, the colony count detection reaches the level of "not detected", which can meet the requirements of product sterility.

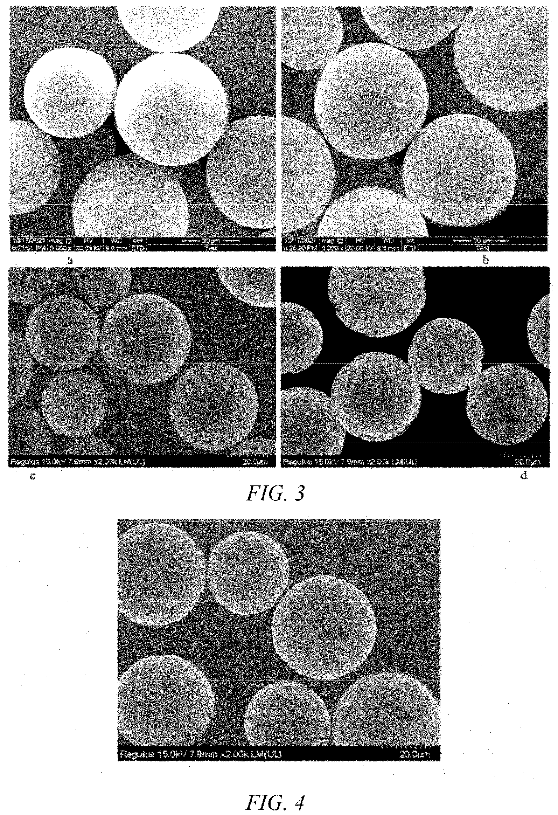

From the analysis of the polydispersity index, crystallinity, roundness, roundness change rate and surface smoothness (observed by microscope and scanning electron microscope) of the PCL microspheres, it was found that: the polydispersity index of PCL microspheres after γ-ray sterilization is increased, and the crystallinity is also increased. Of particular interest is the change in crystallinity; since the measured crystallinity is an average value, which is a result of averaging of high and low crystallinities, it is speculated that uneven crystallinity changes may have occurred within the microspheres; furthermore, local crystallinity increases are likely to cause local volumetric collapse, which leads to the surface of PCL microspheres becoming unsmooth and the formation of certain cracks or small pits. This is also confirmed by scanning electron microscopy (as shown in FIGS. 3a to 3d and 4), wherein FIGS. 3a to 3d show electron microscope photographs of PCL microspheres of 10,000 Da irradiated with γ rays at 15, 20, 30, and 40 kGy. The microspheres irradiated by 15 kGy of γ rays have a roundness value of 0.9892 and a roundness change rate of 0.59%. It can be seen from the electron microscope photograph, FIG. 3a, that a small amount of cracks appear on the microspheres, but the microspheres as a whole do not change much. After irradiation with γ rays at 20 kGy and 30 kGy, the roundness values of the microspheres decrease to 0.9765 and 0.9675, respectively, and the roundness change rates are 1.87% and 2.77%, respectively. As shown in FIGS. 3b and 3c, the changes in the PCL microspheres are more obvious, with multiple cracks and depressions appearing on the surface of the microspheres, but it can be seen from the electron microscope photographs that the overall sphericity of the microspheres has not changed much, and the microspheres still retain a better sphere shape.

Greater changes in the polydispersity index, crystallinity and roundness value of PCL microspheres are found when sterilization is performed by means of irradiation with γ rays at 40 kGy. The roundness value decreases to < 0.96 (0.9547), and the roundness change rate reaches 4.06%. It can also be seen from the electron microscope photograph, FIG. 3d, that a large number of cracks and depressions appear on the surface of the irradiated PCL microspheres, and the microspheres appear to be obviously wrinkled; numerous wrinkles result in changes in the shape of the spheres, and some of the microspheres cannot form a complete sphere. Furthermore, it is known in the art that when microspheres have poor surface smoothness and sphere shape, the rough and irregularly shaped microspheres will be surrounded by macrophages and multinucleated giant cells, leading to capsular fibrosis, inhibition of collagen production, and adverse foreign-body responses.

Moreover, PCL microspheres having a weight-average molecular weight of **40,000** Da are more susceptible to the effects of γ-ray irradiation than PCL microspheres of **10,000** Da. This can be seen from the roundness values and the roundness change rates in Tables **1** and **2,** wherein considering PCL microspheres with different molecular weights, PCL microspheres having a weight-average molecular weight of **10,000** Da, after having been irradiated with γ rays at **30** kGy, have a roundness of **0.9675** and a roundness change rate of **2.77%,** whereas PCL microspheres of **40,000** Da, after having been irradiated with γ rays at **30** kGy, have a roundness of **0.9618** and a roundness change rate of **3.50%.** This is also demonstrated by the observation of electron microscope photographs; FIG. **4** shows the electron microscope photograph of PCL microspheres of **40,000** Da after being irradiated with γ rays at **30** kGy, and it can be seen therefrom that the surface of the microspheres has more cracks and depressions than that of PCL microspheres of **10,000** Da at the same dose (FIG. 3c). These all confirm that high-molecular-weight PCL microspheres are more susceptible to the effects of irradiation.

### Example 4: Sterilizing with β rays to obtain sterile PCL microspheres

The method for sterilizing PCL microspheres by means of irradiation is basically the same as the steps used in Example **3,** with the difference being that the irradiation rays used for the sterilization are β rays (electron accelerator).

Four doses of **15, 20, 30 or 40** kGy were selected for irradiation, and the PCL microspheres, after being irradiated by β rays, were analyzed and detected. In the detection, no colonies were detected at doses of **40** kGy or more.

**Table 3: Sterile PCL microspheres having a weight-average molecular weight of 10,000 Da:**

| **Irradiation dose** | **Colony count** | **Particle size range** | **Polydispersit y index PDI** | **Crystallinity** | **Roundness** | **Roundness change rate** |
|---|---|---|---|---|---|---|
| **15** kGy | **< 10** | Average particle size: **35.41** µm; | **2.1820** | **73.3479%** | **0.9910** | **0.41%** |
| | | Microspheres of less than **20** µm account for **0.75%** | | | | |
| | | Microspheres of **20-25** µm account for **18.61%** | | | | |
| | | Microspheres of **25-50** µm account for 65.75% | | | | |
| | | Microspheres of more than **50** µm account for **14.89**% | | | | |
| **20 kGy** | < **10** | Average particle size: **35.33** µm; | **2.1918** | **73.7378%** | **0.9875** | **0.76%** |
| | | Microspheres of less than **20** µm account for **0.79**% | | | | |
| | | Microspheres of **20-25** µm account for **18.59%** | | | | |
| | | Microspheres of **25-50** µm account for **66.27%** | | | | |
| | | Microspheres of more than **50** µm account for **14.35%** | | | | |
| **30 kGy** | **<5** | Average particle size: **35.25** µm; | **2.1998** | **74.2581%** | **0.9691** | **2.61%** |
| | | Microspheres of less than **20** µm account for **0.85%** | | | | |
| | | Microspheres of **20-25** µm account for **18.61%** | | | | |
| | | Microspheres of **25-50** µm account for **66.50%** | | | | |
| | | Microspheres of more than **50** µm account for **14.04%** | | | | |
| **40** kGy | Not detected | Average particle size: **35.13** µm; | **2.2270** | **76.2773%** | **0.9568** | **3.85%** |
| | | Microspheres of less than **20** µm account for **0.92**% | | | | |
| | | Microspheres of **20-25** µm account for **18.62%** | | | | |
| | | Microspheres of **25-50** µm account for **66.63%** | | | | |
| | | Microspheres of more than **50** µm account for **13.83%** | | | | |

**Table 4: Sterile PCL microspheres having a weight-average molecular weight of 40,000 Da:**

| **Irradiation dose** | **Colony count** | **Particle size range** | **Polydispersit y index PDI** | **Crystallinity** | **Roundness** | **Roundness** |
|---|---|---|---|---|---|---|
| **15** kGy | **< 10** | Average particle size: **37.13** µm; Microspheres of less than **20** µm account for **0.78%** | **1.8673** | **69.1996%** | **0.9901** | **0.66%** |
| | | Microspheres of **20-25** µm account for **14.69%** | | | | |
| | | Microspheres of **25-50** µm account for **70.63%** | | | | |
| | | Microspheres of more than 50 µm account for **13.90%** | | | | |
| **20** kGy | **< 10** | Average particle size: **37.01** µm; | **1.8896** | **69.4893%** | **0.9856** | **1.11%** |
| | | Microspheres of less than **20** µm account for **0.82%** | | | | |
| | | Microspheres of **20-25** µm account for **14.79%** | | | | |
| | | Microspheres of **25-50** µm account for **70.86%** | | | | |
| | | Microspheres of more than **50** µm account for **13.53%** | | | | |
| **30** kGy | **<5** | Average particle size: **36.94** µm; | **1.9026** | **70.0297%** | **0.9662** | **3.06%** |
| | | Microspheres of less than **20** µm account for **0.87%** | | | | |
| | | Microspheres of **20-25** µm account for **14.87%** | | | | |
| | | Microspheres of **25-50** µm account for **71.14%** | | | | |
| | | Microspheres of more than **50** µm account for **13.12%** | | | | |
| **40 kGy** | Not detected | Average particle size: **36.83** µm; | **1.9212** | **73.0384%** | **0.9563** | **4.05%** |
| | | Microspheres of less than **20** µm account for **0.98%** | | | | |
| | | Microspheres of **20-25** µm account for **14.98%** | | | | |
| | | Microspheres of **25-50** µm account for **71.23%** | | | | |
| | | Microspheres of more than **50** µm account for 12.81% | | | | |

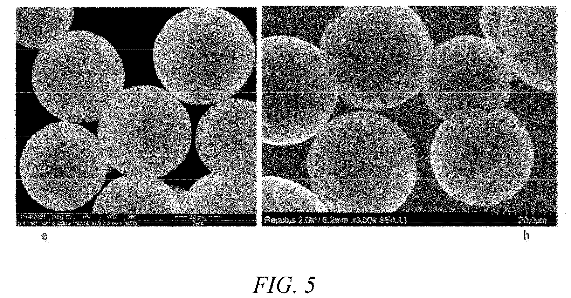

It can be seen from the above results that the colony count of the PCL microspheres is significantly reduced after β-ray irradiation sterilization, and the colony count gradually decreases as the dose increases, wherein both PCL microspheres having a weight-average molecular weight of **10,000** Da and PCL microspheres having a weight-average molecular weight of **40,000** Da are reduced from < 100 (grade) before sterilization to < **10,** < **10,** < **5** and not detected, respectively, which indicates that **β-ray** irradiation can also sterilize PCL microspheres, but its sterilizing effect is not as good as γ rays at the same dose. From the analysis of the polydispersity index, crystallinity, roundness and roundness change rate of the PCL microspheres, it was found that: the polydispersity index of PCL microspheres is increased before and after sterilization, and the crystallinity is also increased. It can be seen from Tables 3-4 that the polydispersity index, crystallinity and roundness of PCL microspheres having a molecular weight of 10,000 Da are all affected by irradiation when the PCL microspheres are sterilized by irradiation with β rays at 30 kGy, with the roundness > 0.96 (0.9691) and the roundness change rate being 2.61%, whereas PCL microspheres having a molecular weight of 40,000 Da show a roundness of > 0.96 (0.9662) and a roundness change rate of 3.06%. It can be seen from FIG. 5a that although the surface of the PCL microspheres having a molecular weight of 10,000 Da also show depressions and grooves, the sphere shape is well maintained, and the overall surface of the spheres is still relatively smooth. Since the effect of irradiation on PCL microspheres having a molecular weight of 40,000 Da is not much different from that on PCL microspheres having a molecular weight of 10,000 Da, and there is only a slight increase in the degree of destruction, it is not shown in the figures for the sake of simplicity.

When PCL microspheres are sterilized by irradiation with β rays at a dose of 40 kGy, it can be seen from Tables 3-4 that both the surface smoothness and the roundness of both PCL microspheres of 10,000 Da and 40,000 Da are greatly affected by irradiation, with the roundness of both being < 0.96 (0.9568, 0.9563), and the roundness change rate reaching 3.85% and 4.05%, respectively. It can be seen therefrom that, similar to the case of γ irradiation, PCL microspheres having a weight-average molecular weight of 40,000 Da are more susceptible to the effects of β-ray irradiation than PCL microspheres of 10,000 Da. It can be seen from FIG. 5b that for PCL microspheres of **10,000** Da, there are obvious depressions and grooves on the surface thereof, and the shape of the spheres has also changed. This is highly unconducive to ensuring the surface smoothness and sphere shape of PCL microspheres and reducing adverse foreign-body responses. Therefore, although the sterilization requirement of "not detected" can be achieved at this dose, this application does not recommend sterilizing PCL microspheres by irradiation with **β** rays at a dose of **40** kGy.

In addition, since β-ray irradiation at 15 and 20 kGy has less effect on PCL microspheres in terms of polydispersity index, crystallinity, roundness, and the roundness change rate as compared to that at 30 kGy, these are not repeated in the specification to avoid repetition.

In summary, the use of β-ray irradiation sterilization is not as effective as γ-ray irradiation sterilization. When β rays at a dose of **15-30** kGy are used for sterilization, the sterilizing effect is unsatisfactory and there is still bacterial residue in the microspheres, and when β rays at a high dose of **40** kGy are used to sterilize PCL microspheres, the roundness and surface smoothness of the microspheres are greatly affected; in summary, the selection of γ rays at a dose of **20-30** kGy is the most favourable for the irradiation sterilization of PCL microspheres having a weight-average molecular weight of **10,000-40,000** Da.

### Example 5: Sterilization of CMC-Na gel

Since irradiation sterilization has a severe effect on the viscosity of CMC-Na, carrier gels based on sodium carboxymethylcellulose (CMC-Na) cannot withstand sterilization by irradiation with rays. Therefore, moist heat steam sterilization, i.e., a high-pressure steam sterilization method, is selected for the sterilization of CMC-Na gel, with the steam pressure being about **101-105** kPa, the temperature being **119-123°C,** for **15-20** min. The specific steps are as follows:
Step **1:** transferring a manufactured CMC-Na gel to a mixing container and placing same in an autoclave; and
Step **2:** performing steam sterilization by using the high-pressure steam sterilization method, wherein the steam pressure is about 101-105 kPa, the temperature is **119-123°C,** for **15-20** min.

### Example 6: Mixing sterile PCL microspheres and CMC-Na gel obtained from sterilization treatment to form sterile beauty product

Step 1, weighing 10 ml of the gel, pouring same into a centrifuge tube, and carefully scraping off the gel adhering to the wall of the measuring cylinder by using a scraper and transferring same to the centrifuge tube until the loss of gel hanging on the wall is minimised;
Step **2,** adding a weighed amount of 3 g of the microspheres to the gel and subjecting same to preliminary stirring by using a glass rod or a thin scraper, scraping the microsphere gel adhering to the stirring rod back to the edge of the centrifuge tube to ensure minimal loss; and
Step **3,** preparing the samples, which have been subjected to preliminary mixing, and stirring and defoaming same by using a vacuum mixing and defoaming machine.

### Example 7: Injecting beauty product into sterilized syringe to form sterile injectable beauty product

A syringe and a needle cap were allowed to enter an LAF laminar air flow device (Laminar Air Flow) in a sterile manner. The beauty product was filled into the syringe by means of perfusion to the correct volume and an end cap was attached to form a sterile barrier. All the mixing and filling steps were performed in a sterile manner in an ISO Class **5** LAF device, and a label was applied to obtain a sterile injectable beauty product which was then stored.

## Claims

1. A beauty product containing sterile PCL microspheres, **characterized by** comprising sterile PCL microspheres, wherein the sterile PCL microspheres are sterilized by irradiation, have a smooth surface, an average particle size range of **26-46** µm and a roundness of ≥ **0.96.**

2. The beauty product containing sterile PCL microspheres as claimed in claim 1, **characterized in that** the sterile PCL microspheres have a roundness of ≥ **0.97.**

3. The beauty product as claimed in claim **1, characterized in that** the sterile PCL microspheres are obtained by means of irradiation sterilization with γ or β rays.

4. The beauty product as claimed in claim **3, characterized in that** the irradiation sterilization is carried out using γ rays, and the irradiation dose is **20** kGy-**30** kGy.

5. The beauty product as claimed in claim **4, characterized in that** the irradiation dose is **20** kGy, 25 kGy, or **30** kGy.

6. The beauty product as claimed in claim **1, characterized in that** in the sterile PCL microspheres, the microspheres with a particle size of **25-50** µm have a percentage of ≥ **65%,** and the microspheres with a particle size of less than **25** µm have a percentage of ≤ **20%.**

7. The beauty product as claimed in claim **6, characterized in that** in the sterile PCL microspheres, the microspheres with a particle size greater than **50** µm have a percentage of ≤ **15%.**

8. The beauty product as claimed in claim **1, characterized in that** the sterile PCL microspheres have a weight-average molecular weight of **8,000-80,000** Da.

9. The beauty product as claimed in claim **8, characterized in that** the sterile PCL microspheres have a weight-average molecular weight of **10,000-40,000** Da.

10. The beauty product as claimed in claim **1, characterized in that** the sterile PCL microspheres have a polydispersity index PDI of **1.5-3.0.**

11. The beauty product as claimed in claim **10, characterized in that** the sterile PCL microspheres have a polydispersity index PDI of **1.8-2.5.**

12. The beauty product as claimed in claim **1, characterized in that** the sterile PCL microspheres have a roundness of ≥ **0.97.**

13. The beauty product as claimed in claim **1, characterized in that** the change rate of the roundness of the PCL microspheres before and after irradiation is ≤ **3.5%.**

14. The beauty product as claimed in claim **1, characterized in that** the change rate of the roundness of the PCL microspheres before and after irradiation is ≤ **2.5%.**

15. The beauty product as claimed in claim **1, characterized by** further comprising a sterile CMC-Na gel resulting from a sterilization treatment by means of a moist heat sterilization method.

16. The beauty product as claimed in claim **1, characterized by** further comprising a sterilized syringe.

17. A method for preparing the beauty product containing sterile PCL microspheres according to claim **1, characterized by** comprising a step of mixing sterile PCL microspheres with a sterile CMC-Na gel resulting from sterilization by means of a moist heat sterilization method until uniform.

18. A method for preparing the sterile PCL microspheres as claimed in claim **1, characterized in that** specific steps for obtaining sterile PCL microspheres by means of irradiation sterilization are as follows:
step **1,** weighing an appropriate amount of PCL microspheres and placing same in a centrifuge tube, and marking the irradiation dose and the type of microspheres on the centrifuge tube;
step **2,** in order to ensure a sterile state and no leakage during detection, sealing the centrifuge tube with a sealing film;
step **3,** putting the centrifuge tube into a packaging box, and pasting a label on the outside of the box to label information about the sterilization dose; and
step **4,** receiving irradiation with γ rays or β rays.

19. The method as claimed in claim **17,** wherein the irradiation dose is **20-30** kGy.

20. The method as claimed in claim **19,** wherein the irradiation dose is **20** kGy, **25** kGy, or **30** kGy.

21. A method for preparing the sterile CMC-Na gel as claimed in claim **17, characterized in that** the specific steps are as follows:
step **1,** transferring the gel to a container to be sterilized, and placing same in an autoclave; and
step **2,** performing steam sterilization by using a high-pressure steam sterilization method, wherein the steam pressure is **101-105** kPa, the temperature is **119-123°C,** for **15-20** min for steam sterilization.

22. The preparation method as claimed in claim **21, characterized in that** the uniform mixing step comprises: performing uniform mixing under vacuum conditions.

23. The preparation method as claimed in claim **21, characterized in that** the uniform mixing step is performed by using a planetary agitator or a vacuum mixing and defoaming machine.

24. A method for preparing the beauty product as claimed in claim **1, characterized in that** the specific steps are as follows:
step **1,** performing irradiation sterilization to obtain sterile PCL microspheres; performing sterilization by means of a moist heat sterilization method to obtain a sterilized CMC-Na gel; and sterilizing a syringe with ethylene oxide;
step **2,** adding the sterile PCL microspheres resulting from the sterilization to the CMC-Na gel and mixing same until uniform; and
step **3,** packaging the uniformly mixed CMC-Na gel loaded with the sterile PCL microspheres into the syringe.

25. A method for preparing the PCL microspheres in claim **1, characterized in that** the specific steps are as follows:
dissolving a PCL material in DCM to form an oil phase; dropwise adding the oil phase to an MC solution under stirring, subjecting same to high-speed shearing by means of a high-speed shear mixer, and stirring same for **2-4** h; and subjecting same to washing with water and centrifugation **3** times, followed by screening and drying to obtain PCL microspheres, wherein the ratio of the oil phase to an aqueous phase is **1** : **5** to **1** : **50;** and the mass concentration of polycaprolactone in the oil phase is **10** mg/ml to **50** mg/ml.

26. The method as claimed in claim **25, characterized in that** after the step of high-speed shearing, there is also a step of ultrasonic treatment.

27. The method as claimed in claim **25, characterized in that** the high-speed shearing is performed at a rotational speed greater than **12,000** r/min for **1-5** min.

28. The method as claimed in claim **25, characterized in that** the ultrasonic treatment is performed for **3-6** min.

29. The method as claimed in claim **25, characterized in that** the stirring is performed at a rotational speed of **800-1,200** r/min for **2-4** h.
